# EUROPÄISCHE PATENTANMELDUNG

(11) **EP 3 552 635 A1**
(43) Veröffentlichungstag der Anmeldung: **16.10.2019**
(21) Anmeldenummer: 18166385.7
(22) Anmeldetag: 09.04.2018
(51) Int. Cl.: A61M 1/10

(54) **KANÜLENSYSTEM UND VERFAHREN ZUR VOLUMENENTLASTUNG EINES HERZENS**

(71) Anmelder: Berlin Heart GmbH, 12247 Berlin (DE)
(72) Erfinder: Menon, Ares, K., 12209 Berlin (DE); Hetzer, Roland, 14057 Berlin (DE)
(74) Vertreter: Pfenning, Meinig & Partner mbB

(57) **Zusammenfassung**

Die vorliegende Erfindung behandelt ein Kanülensystem (500) zur Punktierung des Herzens, mit einer Kanüle (502) und einem Trokar (506), wobei die Kanüle einen Kanülenschaft mit einem herzseitigen Einlass und einem pumpenseitigen Auslass umfasst. Der Trokar besitzt einen in das Lumen der Kanüle einführbaren Trokarschaft mit einer Punktierungsspitze, wobei die Punktierungsspitze die Einlassöffnung der Kanüle vollständig überdecken kann.

## Beschreibung

Gegenstand der vorliegenden Anmeldung ist eine Kanüle zur Entlastung des Herzens, ein Kanülensystem, das eine derartige Kanüle sowie einen Trokar umfasst, ein Herzpumpensystem, das eine Herzpumpe sowie mindestens eine anmeldungsgemäße Kanüle umfasst, und weiterhin ein Verfahren zur Volumenentlastung eines Herzens.

Bei akutem Linksherzversagen nach Myokardinfarkt, Dekompensation einer Herzinsuffizienz oder bei anderen Pathologien der Linksherzfunktion mit Dekompensation entsteht eine Pumpschwäche der linken Herzkammer, die unter anderem drei Effekte hat:
Erstens kommt es zu einer Dilatation des linken Ventrikels mit erhöhter Volumen- und Druckbelastung. Zweitens treten eine Unterversorgung des Körperkreislaufs mit Sauerstoff und nährstoffreichem Blut, eine Übersäuerung des Gewebes (Azidose) sowie ein drohendes Organversagen auf. Drittens treten ein Blutrückstau in der Lungenstrombahn mit einem erhöhten pulmonalen Kapillardruck sowie Lungenhochdruck und ein drohendes Lungenödem auf.

Beim Auftreten des Linksherzversagens werden oftmals intra-aortale Ballonpumpen (IABP), aber auch extrakorporale Herzunterstützungssysteme für eine extrakorporale Membranoxigenation (ECMO) eingesetzt. Der Zugang zu großen Körpergefäßen wird dabei zumeist über die Leistengefäße hergestellt. Hierdurch kann innerhalb weniger Minuten ein suffizienter Kreislauf im Patienten sichergestellt werden. Da bei einem akuten Linksherzversagen häufig ein Lungenstau mit Lungenödem vorliegt, wird ein Oxigenator zur verbesserten Sauerstoffzufuhr in Serie geschaltet. Jedoch ist beim Linksherzversagen das mangelnde Pumpverhalten des linken Ventrikels ursächlich, die schlechte Oxigenierung ist nur sekundär. Dennoch hat sich auch bei der Patientenkohorte mit primärem Linksherzversagen die ECMO durchgesetzt, weil sie schnell und effektiv den marginalen oder fehlenden Kreislauf wieder herstellt. Daher wurde der Begriff der extra-korporalen Lebensunterstützung (Extra Corporal Life Support; ECLS) eingeführt, um diese Patientenkohorte von der reinen ECMO-Lungen-Versagerkohorte abzugrenzen. Bei der ECMO wird eine große Körpervene als Zustrom zur Pumpe bzw. zum Oxygenator und eine große Arterie als Einstrom des Blutes in den Körperkreislauf gewählt. Aufgrund dieser Kanülierungstechnik kommt es nicht zu einer direkten Volumenentlastung des schwer kontraktionsgestörten linken Ventrikels. Somit ist eine eventuelle Verbesserung der linksventrikulären Kontraktilität kaum möglich.

Weitere beschrieben Verfahren zur Entlastung des linken Ventrikels, z.B. über den linken Vorhof mittels eines Katheters oder einer Atrio-Septektomie können keine effiziente Entlastung des linksventrikulären Cavums erzielen. Daher sind die Chancen, dass sich ein Patient alleine durch die ECLS in seiner linksventrikulären Kontraktilität erholt, sehr gering.

Eine effektive Möglichkeit bieten jedoch linksventrikuläre Unterstützungssysteme, so genannte "Left-Ventricular Assist Devices" (LVAD). Diese sind jedoch in der Implantationstechnik komplex und werden in vielen Fällen bei einer akuten Linksherz-Dekompensation nicht sofort implantiert. Zudem kosten LVADs ein Vielfaches der oben genannten ECMO-, IABP- oder ECLS-Pumpensysteme.

Bei den linksventrikulären Unterstützungssystemen ist die Punktierung des Herzens von großer Bedeutung. Zumeist wird hier bei der Implantation der Thorax freigelegt um das Herz zum einen zu punktieren und zum anderen im Bereich der Punktierung einen Naht-Ring zur Befestigung der Pumpe am Herzen zu anzubringen.

Aufgabe der vorliegenden Erfindung ist es, ein effizientes, leicht implantierbares System zur rasch anwendbaren und sicheren Implantation am Herzen zu schaffen, so dass es zu einer raschen Volumenentlastung des linken Ventrikels kommt und damit auch die Kontraktilität des linken Ventrikels verbessert werden kann.

Die Aufgabe wird mit Hilfe der in den Ansprüchen genannten Kanülensysteme sowie einem in Verbindung mit dem Kanülensystem ausgelegten Herzunterstützungssystem gelöst. Ferner wird ein Verfahren zur Volumenentlastung des linksseitigen Herzens offenbart.

Das anmeldegemäße Kanülensystem zur Punktion des Herzens umfasst zumindest eine Kanüle und einen für diese Kanüle ausgelegten Trokar. Die Kanüle besitzt einen Kanülenschaft mit einem herzseitigen Einlass, welcher einen eine Einlassöffnung begrenzenden Einlassrand aufweist. An einem anderen Ende befindet sich ein pumpenseitiger Auslass wobei sich zwischen dem Ein- und dem Auslass ein Lumen erstreckt. Unter einem pumpenseitigen Auslass ist hierbei zu verstehen, dass der Auslass der Kanüle derart ausgebildet ist, dass dieser mit bekannten Pumpensystemen, z.B. KurzzeitZentrifugalpumpen, verbunden werden kann. Insbesondere kann dies in einigen Ausführungsformen bedeuten, dass entweder die Kanüle direkt oder über einen Konnektor an einer Pumpe angeordnet werden kann oder dass ein an der Pumpe angeordneter Konnektor direkt mit der Kanüle verbunden werden kann.

In einigen Ausführungsbeispielen ähnelt die derartige Kanüle den Berlin Heart Excor Kanülen, insbesondere den Berlin Heart Excor Apex Kanülen. So kann die Kanüle beispielsweise aus Silikon gefertigt sein, wobei das Silikon eine Wandungsstärke zwischen 1 und 5 mm betragen kann. Ein alternatives Material ist beispielsweise Polyurethan (PU). Dabei ist das Polyurethan in einigen Ausführungsformen mit einer Beschichtung versehen, beispielsweise um die Risiken zur Thrombenbildung zu verringern.

Ferner umfasst das Kanülensystem einen Trokar. Dieser Trokar besitzt einen vom Auslass des Kanülenschafts in das Lumen einführbaren Trokarschaft mit einer Punktierungsspitze. Die Punktierungsspitze ist dabei bevorzugt derart ausgebildet, dass diese einen Myokard durchstoßen kann, d.h., dass diese den Apex des Herzens im Bereich des linken Ventrikels durchstoßen kann. In einigen Ausführungsbeispielen ist die Punktierungsspitze daher kegelförmig oder annähernd kegelförmig ausgebildet. Auf diese Weise wird das Myokardgewebe nicht lediglich durchstoßen, sondern angeritzt und die Punktierungsspitze kann sich aufgrund der Kegelform langsam durch das Myokard in das linke Ventrikel schieben. Dabei wird das Gewebe des Apex schonend verdrängt. Es findet also keine Myektomie, d.h. keine Resektion von Apexgewebe, sondern lediglich eine Verdrängung des Herzgewebes statt. Dies hat beispielsweise bei Kurzzeitanwendungen den Vorteil, dass sich der Herzmuskel schneller erholen kann (auch nach einer Explantation der Kanüle). Dieser Vorteil kommt insbesondere bei pädiatrischen Anwendungen zur Geltung, da die Verdrängung des Herzmuskels (auch aufgrund der geringeren Größe des Kinderherzens) eine schnellere Erholung als nach einer Myektomie zulässt.

Der Trokarschaft ist anmeldungsgemäß länger als das Lumen der Kanüle, so dass die am Einlass des Kanülenschafts herausragende Punktierungsspitze einem aus dem Auslass hinausragenden Teil des Trokarschafts gegenüberliegt. In einer Punktierungskonfiguration ragt die Punktierungsspitze aus dem Einlass der Kanüle. Ferner besitzt der Trokarschaft einen Umfang und eine Geometrie, so dass der Trokarschaft oder die Punktierungsspitze die Einlassöffnung vollständig überdecken. Dies bedeutet im Wesentlichen, dass der Trokarschaft oder die Punktierungsspitze den Einlass vollständig abdichten, so dass bei in der Punktierungskonfiguration eingeführten Trokar keinerlei Flüssigkeit durch den Einlass des Kanülenschafts in das Lumen treten kann bzw. sich beim gemeinsamen Einführen der Kanüle und dem in der Kanüle angeordneten Trokar sich kein Apexgewebe in einem Hohlraum zwischen dem Trokar und der Kanüle ansammeln kann. Somit wird die Thrombengefahr zu einem späteren Zeitpunkt gegenüber anderen Punktierungsverfahren erhöht. Aufgrund der hier offenbarten Konfiguration der Kanüle und des Trokars ist es möglich, die Kanüle ohne Öffnung des Thorax mit dem Herzen zu verbinden. Hierzu wird zunächst der Trokar in die Kanüle eingeführt und die Punktionskonfiguration geschoben. Anschließend wird das Kanülensystem beispielsweise unterhalb des Rippenbogens oder durch den Interkostalraum zum Apex vorgeschoben, wobei nach Erreichen des richtigen Orts am Herzen die Punktierungsspitze weiter an dem Apex vorgeschoben wird und in das linke Ventrikel eindringt. Das die Punktierungsspitze am größten Umfang im Wesentlichen mit dem Umfang der Innenwand des Einlasses übereinstimmt und die Wandstärke der Kanüle vergleichsweise gering ist gegenüber dem Durchmesser des Lumens der Kanüle kann der Einlass der Kanüle selbst in Verbindung mit dem Trokar in dem Apex durch den Myokard in das linke Ventrikel geschoben werden.

Weitere Ausführungen und Ausführungsbeispiele können den nachfolgenden Beschreibungen und Figurenbeschreibungen entnommen werden.

Es zeigen:
- Fig. 1: eine schematische Darstellung eines Kanülen- bzw. Pumpensystems;
- Fign. 2A und: B eine Ausführungsform eines Kanülensystems mit planarem Einlassrand;
- Fign. 3A und: B eine Ausführungsform eines Kanülensystems mit schrägem Einlassrand;
- Fig. 4: eine Ausführungsform eines Kanülensystems mit gefastem Einlassrand; und
- Figs. 5A und B: verschiedene Ausführungsformen eines Kanülensystems mit Dichtlippe;
- Fig. 6: eine Ausführungsform eines Kanülensystems mit einem expandierbaren Hohlraum;
- Fig. 7: weitere Ausführungsform einer Kanüle;
- Fig. 8: weitere Ausführungsform einer Kanüle;
- Fig. 9a bis 9c: Ausführungsform einer Anordnung einer Druckmessleitung.

Nachfolgend werden zunächst weitere Ausführungen zum Verfahren der Volumenentlastung und weitere Ausführungsformen der Erfindung beschrieben.

Beim anmeldungsgemäßen Verfahren zur Druckentlastung können die hier beschriebenen Kanülensysteme verwendet werden. Nachstehend wird beispielhaft ein Punktions- bzw. Kanülierungsverfahren während der Implantation, sowie ein Explantationsverfahren beschrieben.

Um eine rasche Entlastung der linken Herzkammer zu erreichen, wird mithilfe eines minimal invasiven Zugangs über einer antero-laterale Mini-Thorakotomie die Spitze des Linken Ventrikels (Apex) freigelegt, unterhalb des Rippenbogens oder durch den Interkostalraum (4. oder 5. ICR). (Dies entspricht einem operativen Zugang wie bei einer transapikalen TAVR). Die Kanüle wird mittels einer Seldinger-Technik platziert. Hierbei wird zunächst der Führungsdraht mittels einer Punktionsnadel am Apex angesetzt und durchstößt diesen. Unter radiologischer Durchleuchtung wird der Draht (Pigtail) im LV platziert. Anschließend werden der in die Kanüle eingeführte Trokar auf den Führungsdraht aufgefädelt und zum Apex geführt. Da der Apex bereits durch den Führungsdraht punktiert wurde, verdrängt die Punktierungsspitze nunmehr das Gewebe des Myokards im Bereich des Apex und weitet eine Öffnung auf bis die Öffnung auf die Größe der Kanüle geweitet wurde. Eine Entfernung von vitalem Herzmuskelgewebe (Myektomie) ist somit nicht notwendig. Obgleich hierbei auch Myokardgewebe verletzt wird, wird das Gewebe jedoch im Wesentlichen schonend verdrängt und kann eine spätere Erholung des Gewebes vereinfachen. Durch die Aufweitung des Gewebes kann die Kanüle nunmehr in den Ventrikel vorgeschoben werden bis zum Fixierungsrand (Nahtring), der dann durch Einzelknopfnähte mit dem Herzen verbunden wird. Nachdem die am Ventrikel vorverlegten Nähte mit der Kanüle fixiert worden sind und diese mit dem Herzen selbst blutdicht verbunden ist, kann der Trokar zurückgezogen und somit die Kanüle entlüftet werden, um anschließend passager geklemmt zu werden. Optional können weitere fortlaufende Nähte am Nahtring zur besseren Blutdichtigkeit gesetzt werden.

In weiteren Ausführungsbeispielen können beidseitig der Kanüle Distanzhalter bzw. ein oder mehrere Distanzhalter in den Zwischenrippenraum bzw. Interkostalraum eingebracht werden, um ein eventuelles Abknicken oder eine Kompression der Kanüle durch die Rippen zu verhindern. Alternativ kann die Kanüle mit Wandverstärkungen ausgestattet werden, die eine Kompression durch Rippen verhindert. Anschließend kann die Kanüle entlüftet und beispielsweise mit einer handelsüblichen zentrifugalen Kurzzeitblutpumpe, wie z.B. eine Rota-flow von Marquet, eine Hemopump von Sorin, eine Deltasteam DP3 von Medos/Xenios, verbunden werden. Jedoch kann das System auch beispielsweise mit einer parakorporalen Verdrängerpumpe, wie Berlin Heart Excor, verbunden werden.

Die für das Pumpensystem ausgewählte Pumpe wird ferner mit einer weiteren Kanüle, der sogenannten arteriellen Ausflusskanüle, verbunden. Diese arterielle Kanüle kann entweder perkutan oder chirurgisch an die große Leistenarterie oder chirurgisch an die Arteria Subclava angeschlossen werden. Ferner kann in einigen Ausführungsbeispielen bei einem Patienten, sofern er bereits wegen seiner schlechten Kreislaufsituation an eine ECMO/ECLS angeschlossen ist, zunächst der arterielle Zugang des ECMO/ECLS als Ausflussort benutzt werden, und der venöse Zugang wird nach erfolgreicher Etablierung und Kanülierung des Apex mit dem Kanülensystem entfernt. So kann das Blut aus dem linken Ventrikel über das etablierte System geleitet werden und somit eine direkte Entlastung des linken Ventrikels erreicht (Apexkanüle) und eine Unterstützung des Kreislaufsystems (arterielle Ausflusskanüle) erzielt werden. Somit stellt die Kanüle zusammen mit der Pumpe und der Ausflusskanüle ein Kurzzeit-VAD-System dar, mit einer möglichen Unterstützungszeit bis zu 30 Tage (in Abhängigkeit von der verwendeten Pumpe).

Um die Therapie optimal steuern zu können kann in die Kanüle eine zusätzliche Druckmessleitung integriert sein. An der Spitze der Kanüle mündet diese Druckmessleitung im Ventrikel. Diese Druckmessleitung wird dann auslassseitig mit einem handelsüblichen Druckaufnehmersystem verbunden und kann so an einen üblichen Monitor zur hämodynamischen Überwachung angeschlossen werden. Diese Druckmessung kann kontinuierlich die Messung des LVEDP (linksventrikulären Enddiastolischen Druck) anzeigen. Dies ist kann beispielsweise helfen, die optimale Entlastung des linken Ventrikels zu steuern, z. B. Anpassung (Erhöhung oder Erniedrigung der geförderten Menge Blutvolumen pro Minute). Insbesondere spielt dies eine Rolle bei der Entwöhnung des Patienten vom System, nachdem sich das Herz von der Herzschwäche erholt hat.

Bei einer Erholung der Herzfunktion ist es notwendig den Blutfluss durch die Kanüle bzw. Pumpe schrittweise zu reduzieren. Bei der Reduzierung des Flusses wird im linken Ventrikel mehr Blutvolumen zurück belassen, um zu schauen, ob das Herz die Mehrbelastung schafft ohne dass der Füllungsdruck der linken Herzkammer (LVEDP= left ventricular end diastolic pressure) steigt. Um die Kontraktionsleistung des Herzens unter der Mehrbelastung einschätzen zu können wird also bevorzugt der LVEDP herangezogen. Hat sich die Herzfunktion erholt, kann die Apex-Kanüle und damit das VAD- System wieder über den minimal invasiven Zugang entfernt werden.

Hierfür wird der Apexbereich erneut zugänglich gemacht. Die Kanüle wird geklemmt um keinen Blutfluss mehr über das System zu haben. Da keine Myektomie erfolgt ist, kann über bereits bei der Implantation vorgelegte Filze eine Tabaksbeutel-Naht platziert werden, welche dann die Eintritts-Stelle der Kanüle am Apex während und nach Herausziehen der Kanüle verschließt, indem das umliegende Herzmuskelgewebe zusammengezogen wird. Zusätzlich wird die Kanülierungsstelle mit filzarmierten Nähten gesichert.

In einer Ausführungsform umfasst der Trokar eine umlaufende Dichtlippe, welche vorzugsweise am Übergang zwischen der Punktierungsspitze und dem eigentlichen Trokarschaft angeordnet ist. Diese Dichtlippe ist derart angeordnet, dass sie den Einlassrand zumindest teilweise überdeckt, vorzugsweise den Einlassrand vollständig umläuft und überdeckt. Eine zwischen der Punktierungsspitze und dem Trokarschaft angeordnete Dichtlippe hat unter anderem den Vorteil, dass beim Ausführen der Dichtlippe aus dem Kanülenschaft ein taktiles Feedback erzeugt wird und der Trokar sich deutlich leichter durch das Lumen schieben lässt nachdem die Dichtlippe am Einlass der Kanüle ausgetreten ist. Beim daraufhin folgenden Zurückziehen des Trokarschafts dichtet die Dichtlippe die Einlassöffnung vollständig ab, so dass die Einlassöffnung vollständig überdeckt wird, auch wenn der Trokarschaft einen Umfang besitzt, welcher geringer ist als der Umfang der Innenseite des Lumens. Die Dichtlippe kann dabei in verschiedenen Ausführungsformen unterschiedlich gestaltet sein.

So kann die Dichtlippe beispielsweise kragen- oder flanschförmig am Übergang zwischen der Punktierungsspitze und dem Trokarschaft angeordnet sein. Die Dichtlippe kann jedoch auch eine Verlängerung der Punktierungsspitze bilden, so dass die Dichtlippen eine im Wesentlichen kegelförmige Gestalt der Punktierungsspitze verlängern, d.h. in Richtung des Auslasses und nach Außen hin leicht abstehen.

In einer weiteren Ausführungsform ist der Einlassrand angefast und weist in axialer Richtung zur Einlassöffnung hin eine Fase auf. Diese Ausführungsform ist insbesondere dann vorteilhaft, wenn die Punktierungsspitze (oder auch Punktionsspitze) bündig mit dem Einlassrand abschließt. Hierzu kann in einigen Ausführungsbeispielen die Punktierungsspitze ebenfalls angefast sein und ein Fasenwinkel der Punktierungsspitze und ein Fasenwinkel der Fase des Einlassrandes um weniger als 30° voneinander abweichen, insbesondere um weniger als 20° oder 10° voneinander abweichen und insbesondere im Wesentlichen gleich sind. Mit anderen Worten ist bei ähnlichem Fasenwinkel der Einlassrand als Verlängerung der Punktierungsspitze des Trokars zu sehen. Hierdurch wird ein besonders gewebeschonendes Einführen der Kanüle in den Apex ermöglicht. Als Fasenwinkel bieten sich beispielsweise Winkel größer als 30°, gemessen in der Ebene quer zur Längsachse der Kanüle, an, vorzugsweise Winkel von mehr als 45°, besonders vorzugsweise größer als 60°. Auf diese Weise wird ein schonendes Einführen des Kanülensystems in den linken Ventrikel gewährleistet.

In einer weiteren Ausführungsform weist die Punktierungsspitze des Trokars einen expandierbaren Hohlraum auf, und die Punktierungsspitze überdeckt die Einlassöffnung dann, wenn der Hohlraum in einem expandierten Zustand ist. Hierbei ist es in einigen Ausführungsformen vorgesehen, dass die Punktierungsspitze den Einlassrand im expandierten Zustand des Hohlraums zumindest teilweise, vorzugsweise vollständig überdeckt. Hierbei wird, wie in den anderen Ausführungsbeispielen ausgeführt, eine gute Abdichtung zwischen dem Trokar und der Kanüle erreicht und zugleich ein verbessertes Einführen des Kanülensystems in das linke Ventrikel sichergestellt.

Um den Hohlraum zu expandieren, kann vorgesehen sein, dass am auslassseitigen Ende des Trokars ein Medieneinlass vorhanden ist, über welchen ein Medium in den Hohlraum gefüllt werden kann und diesen expandiert. Als Medien können beispielsweise Kochsalzlösungen infrage kommen oder in anderen Ausführungsbeispielen auch Luft.

In vielen Fällen wird der Trokar bzw. die Punktierungsspitze des Trokars ein hartes Material, wie beispielsweise Stahl, oder einen harten bioverträglichen Kunststoff umfassen. Es ist jedoch auch vorgesehen, dass der Trokarschaft genügend Flexibilität besitzt, um durch den Interkostalraum an den linken Apex geführt zu werden. Hierbei kann der Trokar beispielsweise einen Mantel aus einem flexiblen biokompatiblen Kunststoff besitzen.

In einigen Ausführungsbeispielen ist vorgesehen, dass an der Außenseite des Kanülenschafts in der Nähe des Einlasses ein Naht-Ring zur Verbindung der Kanüle mit dem Herzen angeordnet ist. Dabei ist Naht-Ring beispielsweise stoffschlüssig mit dem Kanülenschaft verbunden. Der Naht-Ring kann beispielsweise aus einem Filz bestehen oder diesen umfassen, so dass das Vernähen auf einfache Art und Weise möglich wird.

In einer weiteren Ausführungsform ist am Trokarschaft eine Trokarmarkierung vorgesehen, welche mit einer an der Kanüle angeordneten Kanülenmarkierung korrespondierend angeordnet werden kann. Das heißt, dass die Trokarmarkierung die Kanülenmarkierung beispielsweise in Deckung oder auf dieselbe Höhe (d.h. quer zur Längsachse des Lumens) angeordnet werden können. Diese Markierungen können derart angeordnet sein, dass anhand eines Indeckungbringens der Markierungen erkennbar ist, wenn die Punktierungsspitze des Trokars die Einlassöffnung überdeckt. Insbesondere kann dies in den Ausführungsbeispielen, in welchen eine Dichtlippe oder eine zum Fasenwinkel der Punktierungsspitze korrespondierende Anfasung des Einlassrandes vorgesehen ist, diejenige Stellung gekennzeichnet werden, in welcher die Punktierungsspitze bündig mit dem Einlassrand abschließt bzw. in welcher die Dichtlippe an bzw. auf dem Einlassrand anliegt und das Lumen der Kanüle abdichtet.

In einer weiteren Ausführungsform ist eine weitere Trokarmarkierung vorgesehen, welche in Bezug auf die Längsachse des Trokarschafts und gegenüber der ersten Trokarmarkierung weiter auslassseitig angeordnet ist. Hierdurch kann erkennt werden, dass, wenn die weitere Trokarmarkierung mit der Kanülenmarkierung in Deckung gebracht wurde, erkennbar ist, dass der Trokar nunmehr zurück in Richtung des Auslasses gezogen werden muss, um eine gute Abdichtung der Einlassöffnung mittels der Punktierungsspitze bzw. des Trokarschafts zu bewerkstelligen.

In einigen Ausführungsbeispielen kann vorgesehen sein, dass die Trokarmarkierung des Trokar ein aus dem Trokarschaft herausragender Anschlag ist und die Kanülenmarkierung entweder ein Auslassrand des Auslasses des Kanülenschafts ist oder im Bereich des Auslasses einen Stopp für den Anschlag bereitstellt.

In einer weiteren Ausführungsform ist vorgesehen, dass im Bereich des Einlassrandes der Kanüle eine Metallhülse oder eine Verbundwerkstoffhülse in den Kanülenschaft eingelassen ist. In einigen Ausführungsformen kann die Metallhülse bzw. die Verbundwerkstoffhülse derart in den Kanülenschaft eingelassen sein, dass diese aus dem Silikon- bzw. Kunststoffmantel der Kanüle herausragt und die unbedeckte Metall- bzw. Verbundwerkstoffhülse den Einlassrand bildet. In anderen Ausführungsbeispielen kann vorgesehen sein, dass die Metallhülse oder die Verbundwerkstoffhülse vollständig in den Kanülenschaft eingelassen sind und zumindest durch eine dünne Materialschicht des Kanülenwerkstoffs überdeckt sind. In dieser Ausführungsform wird zum einen eine gute Biokompatibilität aufgrund des Werkstoffs des Kanülenschafts gewährleistet und zum anderen eine entsprechende Versteifung des Einlassrandes gewährleistet, welche beim Einführen des Kanülensystems in den linken Ventrikel vorteilhaft sein kann.

Wie bereits erwähnt, umfasst das Kanülensystem alternativ oder zusätzlich zu den vorbeschriebenen Merkmalen eine Druckmessleitung, welche am Kanülenschaft, im Lumen des Kanülenschafts, oder innerhalb der Wand des Kanülenschafts geführt wird. Beispiele, wie eine Druckmessleitung an der Kanüle angeordnet wird, lassen sich beispielsweise der internationalen Patentanmeldung PCT/EP2017/076811 entnehmen, welche vollumfänglich zum Bestandteil dieser Anmeldung gemacht wird. Dabei ist die Kanüle eine Druckmessleitung mit einem Druckeinlass und einem Druckauslass, wobei der Druckeinlass der Druckmessleitung herzseitig des Nahtrings angeordnet. Dies bedeutet, dass der Druckeinlass im Wesentlichen zwischen dem Nahtring und dem (Kanülen-)Einlass angeordnet ist. Dabei ist der Querschnitt der Druckmessleitung kleiner als der Querschnitt des Kanülenlumens. Vorzugsweise ist der Querschnitt der Druckmessleitung mindestens fünfmal oder zehnmal kleiner als der Querschnitt des Lumens der Kanüle. Auf diese Weise wird gewährleistet, dass die Druckmessleitung den Durchmesser der Kanüle nur unwesentlich erhöht. Mittels der Druckmessleitung ist es möglich, den Druck innerhalb des linken Atriums dauerhaft zu überwachen, ohne eine gesonderte Druckmessleitung beispielsweise über einen Port zeitweise in die Kanüle einschieben zu müssen. Vorzugsweise ist die Druckmessleitung an einer Außenseite des Kanülenschafts entlang geführt. Dabei kann die Druckmessleitung beispielsweise mit der Außenseite des Kanülenschafts verschweißt oder verklebt sein. In einer weiteren speziellen Ausführungsform ist die Druckmessleitung im Lumen der Kanüle geführt. In einer weiteren speziellen Ausführungsform ist die Druckmessleitung als gesondertes Lumen in der Kanülenschaftwand angeordnet. Die Druckmessleitung ist so konfiguriert, dass sie mit einem externen Druckmesssystem, beispielsweise einem handelsüblichen Blutdruckmesssystem wie den Intensivmonitoren von Philips, HP oder Siemens, verbindbar ist. Hierbei ist der Druckauslass beispielsweise mit einem Adapter ausgestattet, der normiert ist und mit dem gewählten System gekoppelt werden kann. Obgleich in zahlreichen Ausführungsformen die Druckmessleitung mit der Außenseite des Kanülenschafts stoffschlüssig verbunden ist, kann die Druckmessleitung auslassseitig über den Auslass der Kanüle hinausragen und deutlich länger als der Kanülenschaft sein, so dass eine Kopplung an ein externes Druckmesssystem möglich ist.

Alternativ zur oder in Kombination mit der Druckmessleitung kann in der Nähe des Einlasses der Kanüle ein Drucksensor, wie beispielsweise ein mechanisch-elektromagnetischer Sensor, angeordnet sein. Auch ein mit einer Membran versehener Drucksensor kann am Einlass der Kanüle angeordnet werden.
Sollte nach der Implantation des hier vorgestellten Kanülen- bzw. Pumpensystems immer noch ein Sauerstoffproblem vorliegen, so kann ein Oxygenator seriell in die Ausflusskanüle hineingeschaltet werden. Weitere Ausführungsformen und Ergänzungen zum hier vorgestellten Kanülensystem ergeben sich aus den nachfolgenden Ausführungsbeispielen und den Ansprüchen.

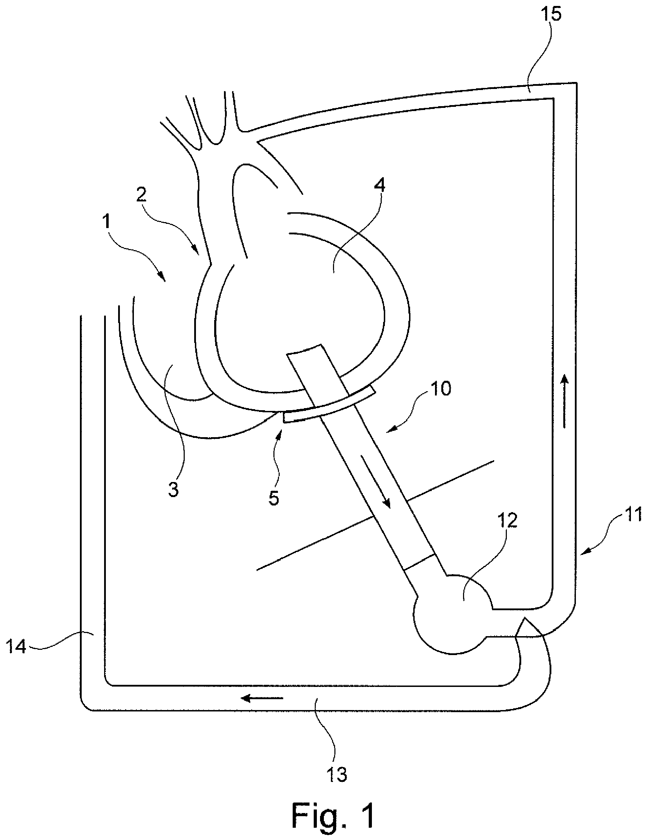

Anhand der Fig. 1 soll schematisch beschrieben werden, in welchem Rahmen ein Kanülensystem bzw. ein Pumpensystem gemäß dieser Anmeldung zur Anwendung kommen kann.

In der Fig. 1 ist ein Kreislaufsystem 1 mit einem Herzen 2 gezeigt, welches unter anderem einen rechten Ventrikel 3 und einen linken Ventrikel 4 besitzt. Am Apex 5 des linken Ventrikels 4 befindet sich eine Öffnung durch welche eine Kanüle 10 als Teil eines Pumpensystems 11 angeordnet ist. Die Kanüle 10 nimmt Blut aus dem linken Ventrikel auf und führt dieses vom herzseitigen Einlass zum pumpenseitigen Auslass in die Pumpe 12, welche mit einer weiteren Kanüle gekoppelt ist, deren Einlass das durch die Pumpe gepumpte Blut aufnimmt und das Blut beispielsweise in der Femoralarterie 14 oder der Subclavia-Arterie 15 wieder in den Kreislauf abgibt. Die Auslasskanüle 13 kann beispielsweise eine Excor-Kanüle bzw. Excor-Gefäßkanüle sein. Sollte ferner eine externe Oxigenation des Blutes gewünscht sein, kann zwischen die Pumpe 12 und die Auslasskanüle 13 ein Oxigenator geschaltet werden. Die Kanüle 10 soll anhand der nachfolgenden Ausführungsbeispiele näher beschrieben werden. Je nach Anwendungsbereich beträgt die Länge der Kanülen 10 bzw. 11 zwischen 20 und 80 cm. Vorzugsweise sind Längen zwischen 30 und 50 cm angedacht. Zwischen dem Einlass und dem Auslass tritt die Kanüle durch die Haut und wird extrakorporal mit der Pumpe 12 verbunden. Bei der Pumpe 12 kann es sich um ein LVAD oder um eine handelsübliche Kurzzeitpumpe handeln.

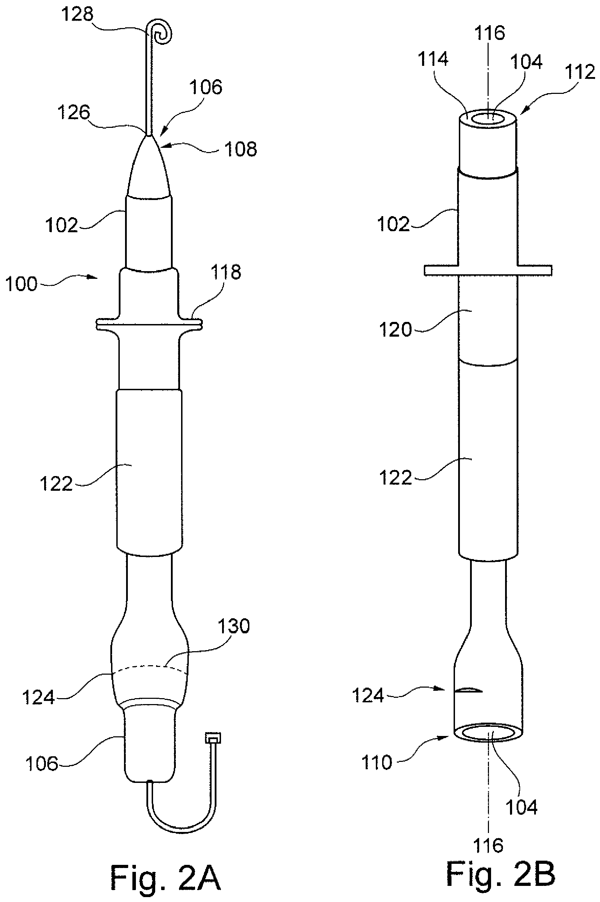

Die Fign. 2A und B zeigen eine Ausführungsform eines Kanülensystems 100. Das Kanülensystem umfasst eine Kanüle 102 und einen durch das Lumen 104 eingeführten Trokar 106 mit einer Punktierungsspitze 108. Das Lumen erstreckt sich dabei zwischen dem pumpenseitigen Auslass 110 und dem herzseitigen Einlass 112. Der Einlass 112 wird durch einen Einlassrand 114 begrenzt, wobei im vorliegenden Ausführungsbeispiel der Fign. 2 der Einlassrand plan ausgeführt ist. Plan bedeutet hierbei, dass der Einlassrand im Wesentlichen in einer Ebene senkrecht zur Längsachse 116 der Kanüle 102 ausgerichtet ist. Die Kanüle weist zwischen dem Einlass 112 und dem Auslass 110 eine Länge von beispielsweise 50 bis 70 cm auf. Die Dicke bzw. Wandstärke der Kanüle beträgt im Bereich des Einlasses 112 beispielsweise 2 mm. Im Bereich des Auslasses 110 kann die Wandstärke geringer ausfallen, um ein Anbinden des Auslasses 110 an eine Pumpe zu vereinfachen. Schematisch ist in der Fig. 2 gezeigt, dass der Auslass 110 gegenüber dem Einlass leicht aufgeweitet ist, um so den Einlass einer Pumpe leichter aufnehmen zu können. Alternativ kann im Bereich des Auslasses 110 ein zusätzlicher Adapter angeordnet sein, welcher das Verbinden mit der Pumpe vereinfacht. An der Außenseite der Kanüle 102 ist ein Naht-Ring 118 angeordnet, welcher mit einem nicht dargestellten Naht-Ring, welcher mit dem Apex des Herzens verbunden werden kann bzw. beim Einführen der Kanüle bereits mit dem Herzen verbunden ist, verbindbar ist. Hierzu ist der Naht-Ring beispielsweise flanschartig ausgebildet und kann beispielsweise aus einem Filz, einem Gewebe oder aus dem Material des Kanülenschafts 120 selbst bestehen bzw. eines dieser Materialien umfassen. Die Länge der Kanüle zwischen dem Einlass 112 und dem Naht-Ring 118 kann beispielsweise 2 bis 5 cm betragen. Diese Länge ist ausreichend um den Naht-Ring im Wesentlichen an der Außenseite des Herzens anzuordnen und den Einlass sicher im Inneren des Ventrikels des Patienten zu verankern. Zwischen dem Naht-Ring 118 und dem Auslass 110 befindet sich ferner eine Armierung 122, welche im Bereich des Durchtritts der Kanüle durch die Haut angeordnet ist und ein Einwachsen der Kanüle im Bereich der Haut begünstigen soll. Hierdurch soll das Infektionsrisiko für den Patienten verringert werden. Obgleich die Kanüle in den Fign. 2 farblos dargestellt ist, kann die Kanüle beispielsweise aus einem transparenten Werkstoff hergestellt sein. Beispielsweise kann die Kanüle im Wesentlichen einen Silikonschaft umfassen. Silikon ist hierbei transparent. Im Bereich des Auslasses ist eine Markierung 124 angeordnet, auf deren Funktion noch genauer eingegangen wird.

Der Trokar 106 weist, wie bereits erwähnt, eine Punktierungsspitze 108 auf. Die Punktierungsspitze erstreckt sich zwischen dem Einlassrand 114 und der Spitze 126 über eine Länge von beispielsweise 3 cm. Dabei läuft die Punktierungsspitze des Trokars vom Einlass 112 zur Spitze 126 hin konisch oder kegelförmig zu. Die Punktierungsspitze weist am einlassseitigen Ende einen Durchmesser auf, welcher identisch oder leicht größer als der Durchmesser der Kanüle im Bereich des Einlasses 112 ohne den eingeführten Trokar ist. Im vorliegenden Beispiel kann der Trokar beispielsweise aus einem biokompatiblen Kunststoff bestehen und die Spitze kann dabei derart geformt sein, dass diese das Gewebe des Myokards im Bereich des Apex zu verdrängen vermag. Im vorliegenden Beispiel ist ferner ein Führungsdraht 128 zu erkennen. Die Kanüle kann beispielsweise mittels einer Seldinger-Technik verlegt werden. Hierbei wird zunächst der Führungsdraht am Apex angesetzt und durchstößt diesen, Anschließend werden der in die Kanüle 102 eingeführte Trokar 106 auf den Führungsdraht aufgefädelt und zum Apex geführt. Da der Apex bereits durch den Führungsdraht punktiert wurde, verdrängt die Punktierungsspitze nunmehr das Gewebe des Myokards im Bereich des Apex und weitet eine Öffnung auf bis die Öffnung auf die Größe der Kanüle geweitet wurde. Obgleich hierbei auch Myokardgewebe verletzt wird, wird das Gewebe jedoch im Wesentlichen verdrängt und kann eine spätere Erholung des Gewebes vereinfachen. Durch die Aufweitung des Gewebes kann die Kanüle nunmehr in den Ventrikel vorgeschoben werden und der Naht-Ring 118 mit einem am Herzen angeordneten Naht-Ring vernäht werden. Nach erfolgter Befestigung der Kanüle am Herzen können der Führungsdraht 128 und der Trokar 106 aus der Kanüle gezogen werden und diese beispielsweise bis zum Anschluss an eine Pumpe passager geklemmt werden. Im eingeführten Zustand des Trokars liegt eine Markierung 130 des Trokars auf derselben Höhe wie die Markierung 124 der Kanüle 102. Hierdurch kann der Kardiologe von außen prüfen, ob der Trokar derart weit in die Kanüle geschoben worden ist, dass die Punktierungsspitze vollständig aus dem Einlass ragt und der Trokarschaft bzw. das auslassseitige Ende der Punktierungsspitze mit dem Einlass bzw. dem Einlassrand abschließt. Da die Punktierungsspitze 108 nunmehr bündig mit dem Einlass 112 abschließt, wird das Einschieben der Kanüle in den Ventrikel gegenüber bestehenden Verfahren deutlich vereinfacht.

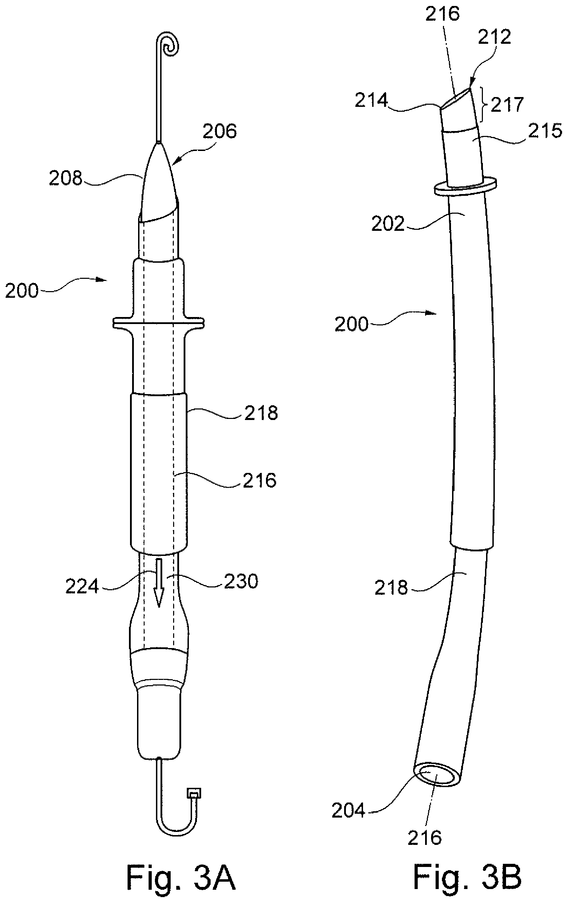

In den Figuren 3 wird ein weiteres Kanülensystem 200 gezeigt. Das Kanülensystem 200 weist ähnlich wie das Kanülensystem 100 eine Kanüle 202 mit einem Lumen 204 auf, um welches ein Trokar 206 eingeführt wurde. Eine Punktierungsspitze 208 des Trokars 206 ragt im eingeführten Zustand aus dem Einlass 212 der Kanüle 202 heraus und überdeckt diesen vollständig. Anders als im Ausführungsbeispiel der Fign. 2 ist der Einlassrand 214 im vorliegenden Beispiel nicht plan, sondern schräg ausgeführt, d.h., der Einlassrand ist in einer Ebene unter einem Winkel von weniger als 90° gegenüber der Längsachse 216 der Kanüle ausgebildet. Aufgrund der schrägen Ausführungsform des Einlassrands kann in manchen Ausführungsbeispielen ein verbesserter Blutfluss bewirkt werden. Wie in Fig. 3B erkennbar ist, ist der zwischen dem Einlassrand 214 in einer Verbindungsnaht 215 ausgebildete obere Einlassabschnitt 217 aus einem anderen Material als der Kanülenschaft 218 gefertigt. Im vorliegenden Beispiel ist der obere Einlassabschnitt aus einem Titan bzw. einer Titanhülse gebildet, welche in den Kanülenschaft eingelassen ist. Das Einlassen kann beispielsweise durch Vergießen oder stoffschlüssiges Verbinden erfolgen. Die Metallhülse, etwa aus Titan, kann beispielsweise verbesserte biokompatible Eigenschaften aufweisen. In den Fign. 3 ist ferner eine alternative Form einer Markierung 224 erkennbar, welche mit einer darunterliegenden Markierung 230 des Trokars in Deckung gebracht wurde. Die Markierung ist hierbei keine reine quer zur Längsachse 216 ausgebildete Markierung sondern ein zweidimensionales Piktogramm. Dadurch, dass der Trokar ein zum Piktogramm der Kanüle korrespondierendes Piktogramm aufweist, kann durch das Indeckungbringen der Markierung bewirkt werden, dass die Punktierungsspitze in einer bestimmten Orientierung durch den Einlass ragt und so auch ein Überdecken des Einlasses bei einer schrägen Ausführungsform des Einlassrandes 214 erreicht werden.

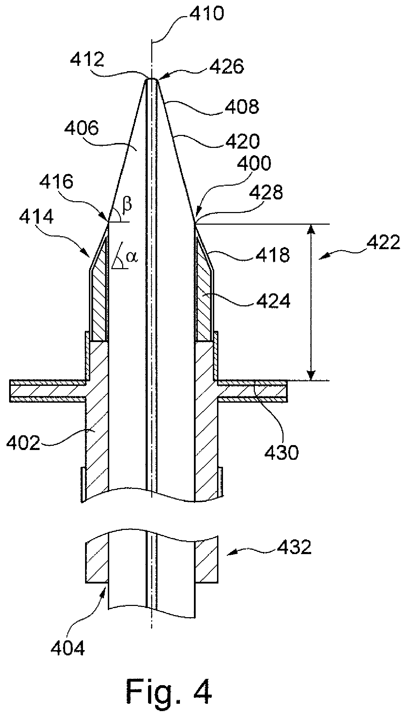

Auch die Fig. 4 zeigt ein Kanülensystem. Das Kanülensystem 400 umfasst neben der Kanüle 402 einen durch das Lumen 404 der Kanüle 402 geschobenen Trokar 406 mit einer Punktierungsspitze 408. Ferner umfasst der Trokar eine entlang der Längsachse 410 der Kanüle (sowie des Trokars) eine Öffnung 412, durch welche ein Führungsdraht geschoben werden kann, um das Kanülensystem mit Hilfe der Seldingertechnik zu implantieren. Der Einlass 414 der Kanüle 402 umfasst einen Einlassrand 416, der im Wesentlichen quer zur Längsachse 410 verläuft, jedoch, anders als das Ausführungsbeispiel der Fign. 2 eine Fase 418 aufweist. Die Fase erstreckt sich dabei über eine Länge von ca. 4 mm. Der Fasenwinkel α beträgt dabei, quer zur Längsachse 410 betrachtet, 75°. Durch diese kegelstumpfartige Zuspitzung des Einlassrands und eine entsprechend ausgebildete Punktierungsspitze 408, welche unter dem gleichen Fasenwinkel β = 75° zuläuft, wird der Einlassrand 416 nunmehr als Verlängerung der Punktierungsspitze 408 ausgebildet, sofern der Trokar korrekt innerhalb der Kanüle platziert wird. Die entsprechende Platzierung kann beispielsweise über die in den Fign. 2 oder 3 beispielhaft genannten Markierungen bewirkt werden. Dadurch, dass die Oberfläche 420 der Punktierungsspitze im Wesentlichen stetig mit der Phase 418 überlappt, und der Trokar bzw. die Punktierungsspitze die Einlassöffnung vollständig überdeckt, wird ein besonders schonendes Einführen der Kanüle in den Apex bzw. in das Ventrikel ermöglicht. Um die Geometrie der Phase präzise herstellen zu können, kann beispielsweise der obere Einlassabschnitt 422 eine Titanhülse 424 umfassen, welche beispielsweise mittels Fräsen oder Ätzen angefast wurde. Die Titanhülse 424 kann dabei vollständig mit dem Silikonmaterial der verbleibenden Kanüle 402 umgossen werden oder kann sich aus dem oberen Teil der Silikonkanüle erstrecken. Obgleich in dem vorliegenden Beispiel die Fasenwinkel α und β im Wesentlichen identisch sind, können diese auch voneinander abweichend gewählt werden. Auch können andere Geometrien, wie beispielsweise ein vom oberen Ende der Spitze 426 der Punktierungsspitze 408 zum unteren Ende 428 der Punktierungsspitze stetig zunehmender Winkel β gewählt werden, so dass die Punktierungsspitze im Wesentlichen in der Spitze spitzer ist als im Bereich des Einlassrandes. Der Einlassrand 416 ist dabei im Wesentlichen 4 cm von einem aus einem Textil gebildeten Naht-Ring 430 entfernt. Die Länge der Kanüle 402 (und entsprechend die Länge des Trokars) zwischen dem Einlass 416 und dem Auslass 432 beträgt beispielsweise 65 cm. Der Trokar kann entsprechend 10 bis 20 cm länger sein.

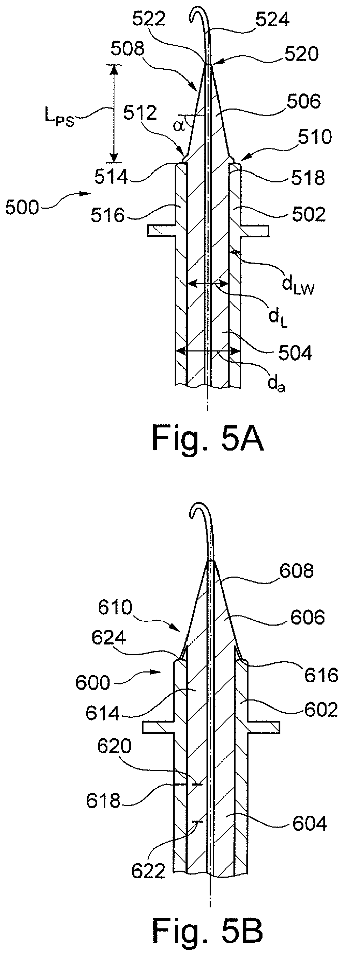

Die Fign. 5 zeigen jeweils ein Kanülensystem, bei welchem der Trokar eine Dichtlippe am unteren Ende der Punktierungsspitze umfasst.

Das Kanülensystem 500 umfasst eine Kanüle 502, in dessen Lumen 504 ein Trokar 506 mit einer Punktierungsspitze 508 angeordnet ist. Am unteren Rand 510 der Punktierungsspitze befindet sich eine Dichtlippe 512, welche den Einlassrand 514 des Einlasses 516 umlaufend überdeckt. Das Lumen 506 weist einen Durchmesser dₗ auf, die Wandstärke d_{lw} beträgt 3 mm. Am unteren Ende 510 der Punktierungsspitze ist die Dichtlippe 512 derart angeordnet, dass diese sich flanschartig aus der Punktierungsspitze heraus erstreckt und ca. 1,5 mm über den inneren Rand 518 des Einlassrandes 514 hinausragt. Hierdurch wird der Einlass 516 vollumfänglich verschlossen und Fremdkörper können nicht in das Lumen 506 eindringen. Die Punktierungsspitze 508 weist eine Länge L_{PS} von 30 mm auf. Die Punktierungsspitze 508 läuft dabei unter einem Winkel α = 80° vom unteren Ende 510 der Punktierungsspitze bis zum oberen Ende 520 konisch zu. Im Inneren des Trokars befindet sich ein Lumen 522, durch welches ein Führungsdraht 524 gefädelt sein kann. Der wulstförmige Rand in Form der Dichtlippe 512 besteht aus einem vergleichsweise weichen Material, so dass der Trokar vollständig durch das Lumen 506 in Richtung des Auslasses gezogen werden kann, und umgekehrt auch vom in der Fig. 5A nicht dargestellten Auslass in das Lumen 506 eingeführt werden kann, und so weit ausgeführt werden kann, dass die Dichtlippe 512 am Einlassrand 514 voll umfänglich anliegt und diesen überdeckt. Da die Dichtlippe eine etwas geringere Fläche als die durch den äußeren Durchmesser des Einlassrandes 514 definierte Fläche abdeckt, ist der äußere Durchmesser dₐ der Kanüle leicht größer als der Durchmesser der Punktierungsspitze 508 im Bereich der Dichtlippe 512. Aufgrund des Winkels α der Punktierungsspitze wird jedoch das Myokardgewebe weit genug aufgeweitet, so dass die Kanüle inklusive der Dichtlippe eingeführt werden kann. Im vorstehenden Beispiel ist der Kanülenschaft beispielsweise aus Silikon gefertigt. Der Trokar kann beispielsweise aus einem biokompatiblen Polyurethan hergestellt sein, wobei dieses im Bereich der Dichtlippe die Innenwand der Silikonkanüle aufzuweiten vermag, d.h. der Trokar 506 ändert seine Form nicht, wohingegen die Innenwand der Silikonkanüle 502 beim Durchführen des Trokars durch das Lumen 506 nach außen gedrängt wird. Um eine entsprechende richtige Anordnung des Trokars zum Einlassrand 514 zu gewährleisten, kann beispielsweise auf eine Markierung zurückgegriffen werden. Neben dem bereits in den Fign. 2 und 3 erwähnten Markierungen kann zusätzlich eine weitere Trokarmarkierung vorhanden sein, welche markiert, dass der Trokar zu weit aus dem Einlass geschoben wurde und nunmehr zurückgezogen werden muss. Dies kann beispielsweise eine weitere proximal der Markierung der Fign. 2 oder 3 angeordnete Trokarmarkierung sein.

In der Fig. 5B ist ein alternatives Kanülensystem 600 gezeigt, welches neben der Kanüle 602 mit dem Kanülenlumen 604 einen Trokar 606 mit einer Punktierungsspitze 608 umfasst. Auch dieses Ausführungsbeispiel weist eine Dichtlippe 610 auf. Die Dichtlippe ist dabei derart gewählt, dass diese sich im Wesentlichen der Form der Punktierungsspitze 608 anpasst und diese im unteren Bereich 612 verlängert. Die Dichtlippe 610 ist somit kragenartig ausgebildet. Wird der Trokar 606 nun vom nicht in der Fig. 5B gezeigten Auslass durch das Kanülenlumen 604 in Richtung des Einlasses geschoben, ist der Kragen 610 an die Außenseiten des unterhalb der Punktierungsspitze 608 gelegenen Trokarschafts 614 angelegt und kann so einfach durch das Lumen geschoben werden. Der Kragen muss zunächst voll umfänglich aus dem Lumen 604 ausgeführt werden und der Trokar anschließend leicht in Richtung des Auslasses zurückgezogen werden, um den nun entfalteten Kragen (wie in der Fig. 5B dargestellt) zu entfalten, und nunmehr mit dem Einlassrand 616 in Deckung zu bringen. Somit wird eine Abdichtung des Einlassrandes durch die Dichtlippe 610 gewährleistet. Zu diesem Zweck weist die Kanüle eine Markierung 618 auf und der Trokar eine erste Markierung 620 und eine zweite Markierung 622. Zunächst wird der Trokar derart durch das Lumen geschoben, bis die Markierung 622 mit der Markierung 618 in Deckung liegt. Auf diese Weise ist für den Bediener klar erkennbar, dass der Kragen 610 vollständig aus dem Lumen ausgeführt wurde und sich somit entfalten konnte. Anschließend wird der Trokar zurückgezogen, bis die Markierung 620 mit der Markierung 618 in Deckung ist, so dass für den Bediener erkennbar ist, dass der untere Rand 624 der Dichtlippe 610 mit dem Einlassrand 616 in Berührung ist und diesen somit sicher abdeckt, so dass sich kein Gewebe im Lumen 604 der Kanüle 602 ansammeln kann.

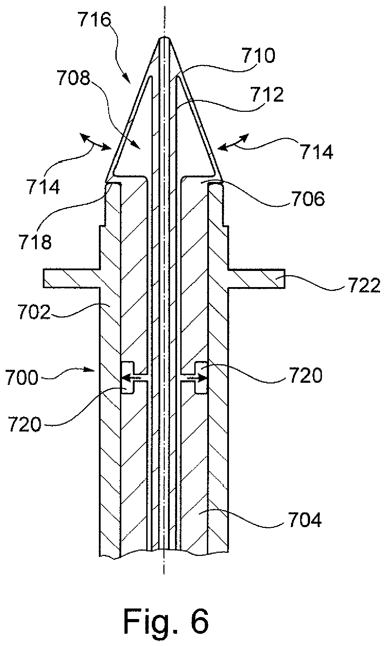

In der Fig. 6 ist ein weiteres Ausführungsbeispiel des Kanülensystems 700 dargestellt. Die Kanüle 702 besitzt ein Lumen 704, durch welches der in der Fig. 6 dargestellte Trokar 706 eingeführt werden kann. Der Trokar 706 weist einen expandierbaren Hohlraum 708 auf, welcher durch eine Außenwand 710 und eine Innenwand 712 begrenzt wird. Schematisch dargestellt ist ein Hohlraumeinlass 714, durch welchen ein Medium, wie beispielsweise eine Kochsalzlösung oder Luft, in den expandierbaren Hohlraum 708 eingefüllt werden kann. Wie anhand der Pfeile 714 angedeutet, wird beim Expandieren des Hohlraums 708 eine Punktierungsspitze 716 geformt, bei welcher der Außendurchmesser im Bereich des Einlassrandes 718 zunimmt und diesen somit überdeckt. Auch hierdurch wird ein sicheres Abdecken des Lumens 704 bewirkt, und es kann sich kein Gewebe zwischen der Punktierungsspitze und dem Kanülenschaft 702 sammeln. Um zusätzlich einen Kraftschluss des Trokars innerhalb der Kanüle zu bewirken, kann der Hohlraum weitere Seitenkammern 720 umfassen, welche den Trokar an die Innenwand des Kanülenschafts anpressen.

Nachdem der Trokar zum Punktieren des Apex verwendet, die Kanüle in den Ventrikel eingeführt und der Naht-Ring 722 mit einem entsprechenden Naht-Ring am Herzen verbunden wurde, kann über den Hohlraumeinlass 714 das Medium abgelassen werden, der Trokar bzw. der Hohlraum zieht sich zusammen und der Trokar auf einfache Art und Weise durch das Lumen 704 herausgezogen werden. Anschließend wird die Kanüle passager geklemmt.

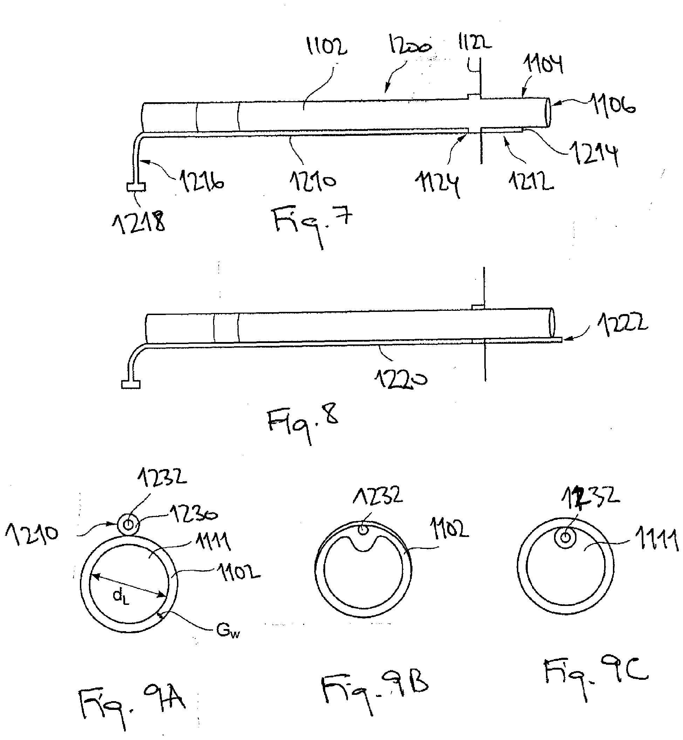

Anhand der Fig. 7 soll eine weitere Variante einer Kanüle beschrieben werden. Die in der Fig. 7 dargestellte Kanüle 1200 umfasst, unter anderem, eine Druckmessleitung 1210, welche sich entlang des Kanülenschafts 1102 erstreckt. Die Einlasskonfiguration der Kanüle, wie beispielhaft in den vorhergehenden Ausführungsbeispielen dargestellt, ist in den nachstehenden Figuren nicht explizit dargestellt, um hier auf die Druckmessleitung einzugehen. Die Ausführungsbeispiele der Figuren 1 bis 6 können also mit den Ausführungsbeispielen der Figuren 7 bis 9 kombiniert werden.

Die Druckmessleitung 1210 besitzt einen Druckmessleitungseinlass 1212 mit einer distalen Öffnung 1214, welcher proximal der distalen Öffnung 1106, jedoch distal des distalen Endes einer Drainagekorböffnung 1110 liegt. Der Drainagekorb ist optional. Der Druckmessleitungsauslass 1216 liegt proximal der proximalen Öffnung 1114 der Kanüle und umfasst einen Adapter 1218, welcher zum Anschluss an ein externes Druckmesssystem ausgebildet ist. Beispielsweise kann es sich bei dem Anschluss um einen Snapfit-Connector oder einen Luerverschluss zum Anschluss an ein externes Druckmesssystem handeln. Derartige Adapter sind im Stand der Technik hinlänglich bekannt. Mittels der Druckmessleitung 1210, welche in die Kanüle 1200 integriert ist, wird erreicht, dass zu jedem Zeitpunkt der Druck im Ventrikel gemessen werden kann. Hierdurch entfällt das Einfädeln einer zusätzlichen Druckmessleitung in die Kanüle selbst, welche in regelmäßigen oder unregelmäßigen Abständen durchgeführt werden muss, um die Druckverhältnisse im Ventrikel zu ermitteln. Im vorliegenden Beispiel der Fig. 7 verläuft die Druckmessleitung 1210 außerhalb des Kanülenschafts 1102 und ist an diesem stoffschlüssig befestigt. Die Druckmessleitung 1210 kann mit der Außenwand des Kanülenschafts 1102 entweder verklebt oder verschweißt sein. Die Druckmessleitung 1210 wird dabei durch eine Öffnung 1124 oder einen Ausschnitt des Nahtrings 1122 geführt, so dass sichergestellt wird, dass der Druckmessleitungseinlass 1212 innerhalb des Ventrikels zum Liegen kommt. Obgleich im vorliegenden Ausführungsbeispiel die distale Öffnung 1214 des Druckmessleitungseinlasses 1212 proximal der distalen Öffnung 1106 liegt, kann, wie in der Fig. 8 gezeigt, in einem anderen Ausführungsbeispiel eine distale Öffnung 1224 eines Druckmessleitungseinlasses 1222 distal der distalen Öffnung 1106 der Kanüle liegen. In anderen Ausführungsbeispielen kann die distale Öffnung des Druckmessleitungseinlasses bündig mit der distalen Öffnung 1106 der Kanüle abschließen.

Anhand der Figuren 9A bis 9C sollen die verschiedenen Positionierungen der Druckmessleitung im Querschnitt dargestellt werden. In der Fig. 9A ist die Wand des Kanülenschafts 1102 mit der Wanddicke G_{w} dargestellt. Die Wanddicke hat vorzugsweise eine gleichbleibende Dicke zwischen 1 bis 5 mm. Das Lumen 1111 der Kanüle 1100 besitzt einen Durchmesser D_{L} von 0,3 bis 2 cm. Die Druckmessleitung 1210 ist außerhalb des Lumens 1111 angeordnet und weist eine separate Leitungswand 1230 auf. Die Leitungswand kann dabei eine vorzugsweise konstante Wandstärke zwischen 0,5 bis 3 mm besitzen. Die Flüssigkeit, das heißt im vorliegenden Beispiel Blut, kann durch die distale Öffnung in das Lumen 1232 eintreten, bis zum Adapter gelangen und so einer Druckmessung mittels eines Druckmessers im externen Druckmesssystem zugeführt werden. Alternativ kann innerhalb der Druckmessleitung ein Druckmesssensor angeordnet werden. Hierbei kann es sich beispielsweise um eine in der Druckmessleitung 1210 angeordnete Membran handeln, wobei die auf die Membran wirkende Kraft in einen Druck umgerechnet werden kann. Hierzu muss die Auslenkung bzw. die auf die Membran wirkende Kraft an ein externes Auswertesystem weitergereicht werden. Das Weiterreichen kann beispielsweise mittels einer elektrischen Leitung, die innerhalb der Wand 1230 verläuft, vorgenommen werden. Um jedoch eine hohe Flexibilität zu gewährleisten, wird die eigentliche Druckmessung oftmals im externen Druckmesssystem stattfinden.

In der Fig. 9B ist das Lumen 1232 in die Wand des Kanülenschafts 1102 integriert. In der Fig. 9C ist die Druckmessleitung (ähnlich wie in der Fig. 9A) separat, das heißt nicht innerhalb der Wand des Kanülenschafts 1102, angeordnet, verläuft jedoch innerhalb des Lumens 1111. Auch hier kann die Druckmessleitung stoffschlüssig mit der Wand des Kanülenschafts 1102 verbunden sein.

## Patentansprüche

1. Kanülensystem zur Punktion oder Volumenentlastung des Herzens, umfassend eine Kanüle und einen Trokar,
wobei die Kanüle einen Kanülenschaft mit einem herzseitigen Einlass, welcher einen eine Einlassöffnung begrenzenden Einlassrand aufweist, und einem pumpenseitigen Auslass und einem sich zwischen dem Einlass und dem Auslass erstreckenden Lumen umfasst; und
wobei der Trokar einen vom Auslass in das Lumen einführbaren Trokarschaft mit einer Punktierungsspitze besitzt und der Trokarschaft länger als das Lumen ist, so dass in einer Punktierungskonfiguration die Punktierungsspitze aus dem Einlass der Kanüle ragt, und
der Trokarschaft oder die Punktierungsspitze die Einlassöffnung vollständig überdeckt.

2. Kanülensystem nach einem der vorhergehenden Ansprüche, wobei die Punktierungsspitze eine umlaufende Dichtlippe aufweist, welche den Einlassrand zumindest teilweise überdeckt.

3. Kanülensystem nach einem der vorhergehenden Ansprüche, wobei sich der Einlassrand in axialer Richtung zur Einlassöffnung eine Fase aufweist und die Punktierungsspitze bündig mit dem Einlassrand abschließt.

4. Kanülensystem nach Anspruch 3, wobei die Punktierungsspitze angefast ist und ein Fasenwinkel der Punktierungsspitze und ein Fasenwinkel der Fase des Einlassrandes um weniger als 30° voneinander abweichen, insbesondere um weniger als 20° oder 10° voneinander abweichen und insbesondere im Wesentlichen gleich sind.

5. Kanülensystem nach einem der vorhergehenden Ansprüche, wobei zumindest die Punktierungsspitze des Trokars einen expandierbaren Hohlraum aufweist, und die Punktierungsspitze die Einlassöffnung vollständig überdeckt, wenn der Hohlraum in einem expandierten Zustand ist.

6. Kanülensystem nach Anspruch 5, wobei die Punktierungsspitze den Einlassrand zumindest teilweise überdeckt, wenn der Hohlraum im expandierten Zustand ist.

7. Kanülensystem nach einem der Ansprüche 5 oder 6, wobei der Trokarschaft einen mit dem expandierbaren Hohlraum verbundenen Hohlraumeinlass aufweist, so dass der Hohlraum über den Einlass expandierbar ist.

8. Kanülensystem nach einem der vorhergehenden Ansprüche, wobei an einer Außenseite des Kanülenschafts ein Nahtring zur Verbindung der Kanüle mit dem Herzen angeordnet ist und der Auslass derart konfiguriert ist, dass der Auslass mit einer Pumpe verbindbar ist.

9. Kanülenssystem nach einem der vorhergehenden Ansprüche, wobei der Trokar an seinem Trokarschaft eine Trokarmarkierung besitzt und die Kanüle eine Kanülenmarkierung besitzt, und die Trokarmarkierung und die Kanülenmarkierung derart zueinander angeordnet werden können, dass erkennbar ist, wenn die Punktierungsspitze des Trokars die Einlassöffnung überdeckt.

10. Kanülensystem nach Anspruch 9, wobei eine weitere Trokarmarkierung vorhanden ist, welche gegenüber der Kanülenmarkierung derart angeordnet werden kann, dass erkennbar ist, dass der Trokar bis zur Trokarmarkierung in Richtung des Auslass gezogen werden muss.

11. Kanülensystem nach einem der Ansprüche 8 bis 10, wobei die Trokarmarkierung des Trokar ein Anschlag und die Kanülenmarkierung ein Auslassrand des Kanülenschafts ist.

12. Kanülensystem nach einem der vorhandenen Ansprüche, wobei im Bereich des Einlassrandes der Kanüle eine Metallhülse oder eine Verbundwerkstoffhülse in den Kanülenschaft eingelassen ist.

13. Kanülensystem nach einem der vorhergehenden Ansprüche, wobei die Punktierungsspitze derart ausgebildet ist, dass das Myokard durchstoßbar ist.

14. Kanülensystem nach einem der vorhergehenden Ansprüche, wobei der Einlassrand gegenüber einer quer zur Längsachse des Lumens des Kanülenschafts liegenden Ebene schräg verläuft.

15. Kanülensystem nach einem der Ansprüche 8 oder einem auf den Anspruch 8 bezogenen Ansprüche, wobei die Kanüle eine Druckmessleitung mit einem Einlass und einem Auslass umfasst, wobei der Einlass der Druckmessleitung herzseitig des Nahtrings angeordnet ist.

16. Kanülensystem nach Anspruch 15, wobei die Druckmessleitung an der Außenseite des Kanülenschafts entlang geführt oder im Lumen der Kanüle geführt ist oder in einer Kanülenwand geführt ist.

17. Pumpensystem, welches eine Herzpumpe, eine Einlasskanüle und eine Auslasskanüle bzw. Ausflusskanüle umfasst, wobei die Einlasskanüle Teil eines Kanülensystems nach einem der vorhergehenden Ansprüche ist.

18. Verfahren zur Kanülierung des linksseitigen Herzens, umfassen die folgenden Schritte:
- Punktion des Apex des Herzens mit einem Führungsdraht;
- Auffädeln eines Kanülensystems, umfassend eine Kanüle und einen Trokar, auf den Führungsdraht;
- Einführen des Kanülensystems bis zum Apex und Punktieren des Apex mittels einer Punktierungsspitze des Trokars;
- Verdrängen des Myokard mittels der Punktierungsspitze des Trokars und Einführen des Einlasses der Kanüle in den Ventrikel;
- und vorzugsweise Vernähen eines Nahtrings der Kanüle mit der Außenwand des Herzens.
